# EUROPEAN PATENT APPLICATION

(11) **EP 4 759 267 A1**
(43) Date of publication of application: **17.06.2026**
(21) Application number: 24876601.6
(22) Date of filing: 10.10.2024
(51) Int. Cl.: A61F 2/24

(54) **PROSTHETIC VALVE STENT, AND PROSTHETIC VALVE AND RELEASE METHOD THEREFOR**

(30) Priority: 13.10.2023 CN 202311331926
(71) Applicant: Mitrassist Lifesciences Limited, Shanghai 201807 (CN)
(72) Inventor: WANG, Zehui, Shanghai 201807 (CN); SHEN, Bin, Shanghai 201807 (CN); ZHU, Jing, Shanghai 201807 (CN); ZHONG, Wei, Shanghai 201807 (CN)
(74) Representative: Vogelbruch, Keang
(86) International application number: PCT/CN2024/123986
(87) International publication number: WO 2025/077789

(57) **Abstract**

An prosthetic valve stent (100), and an prosthetic valve and a release method therefor, which belong to the field of medical technology. The prosthetic valve stent (100) comprises a first stent (10) and a second stent (20), wherein the first stent (10) comprises a first stent body (11) and a first positioning portion (12) which is configured to position with a cardiac valve annulus first side (201), the first stent body (11) being provided with a flow channel (30) for blood to flow through, and the first positioning portion (12) radially extending outward from the first stent body (11); and the second stent (20) comprises a connection portion (21), which is located radially outside the first stent body (11) and connected to the first stent body (11), and a second positioning portion (22), which is configured to position with the cardiac valve annulus second side (202). Such a mitral valve stent reduces the difficulty of releasing the prosthetic valve and improves the convenience of the release operation.

## Description

### CROSS-REFERENCE TO RELATED APPLICATIONS

The present invention claims priority to Chinese Patent Application No. 202311331926.4 filed on October 13, 2023 with the Chinese Patent Office, and entitled "PROSTHETIC VALVE STENT, AND PROSTHETIC VALVE AND RELEASE METHOD THEREFOR", the entire contents of which are incorporated herein by reference.

### TECHNICAL FIELD

The present invention belongs to the field of medical technology, and specifically relates to a prosthetic valve stent, a prosthetic valve, and a release method.

### BACKGROUND ART

Mitral stenosis or regurgitation is one of the common valvular diseases. Due to congenital abnormalities or acquired pathological lesions, the mitral valve cannot close completely during the contraction of the left ventricle. This causes a portion of the blood flowing from the left atrium into the left ventricle to flow back into the left atrium, leading to a series of pathological changes and clinical symptoms in the heart. In severe cases, it can lead to heart failure or even death. Mitral valve replacement surgery improves mitral regurgitation by implanting a prosthetic valve to replace the native mitral valve that has lost function due to the disease, thereby alleviating clinical conditions caused by mitral stenosis or reconstructing the mitral valve.

Most existing mitral valve replacement stents adopt a cylindrical stent shape. After anchoring, they are supported by the radial support force provided by the elasticity of the stent itself. Furthermore, the structures of existing valve stents are mostly single-layer stents, although double-layer stents also exist, for example, a transapical implantable mitral valve device disclosed in Patent No. CN108578016B. Although the stent of this mitral valve device is a double-layer stent, both of its anchoring points are located on the outer layer of the valve stent, and the mitral valve prosthesis is anchored within the body via the outer layer of the valve stent. During the release of the mitral valve prosthesis, the release can only be completed after the target location is precisely positioned, resulting in high difficulty in positioning.

### SUMMARY

Embodiments of the present invention provide a prosthetic valve stent, a prosthetic valve, and a release method, which reduce the difficulty of releasing the prosthetic valve and improve the convenience of the release operation.

Embodiments of the present invention provide a prosthetic valve stent. The prosthetic valve stent includes a first stent and a second stent. The first stent includes a first stent body and a first positioning portion configured to be positioned with a first side of a heart valve annulus. The first stent body defines a flow channel for blood flow, and the first positioning portion extends radially outward from the first stent body. The second stent includes a connection portion located on a radial outer side of the first stent body and connected to the first stent body, and a second positioning portion configured to be positioned with a second side of the heart valve annulus.

In the present embodiment, by adopting a double-layer stent structure for the prosthetic valve stent and designing the positioning structure of the prosthetic valve stent as a split-type structure, a first positioning portion and a second positioning portion are respectively provided on the first stent and the second stent. In this way, during the release of the prosthetic valve, the second stent can be released first. At this time, the first stent has not yet been fully released, and the position of the second stent can be adjusted according to the actual situation. After the second positioning portion of the second stent is positioned on the second side of the heart valve annulus, the first stent is then released to complete the release of the prosthetic valve stent. Thus, before releasing the second stent, after the sheath enters the target position, it is not necessary to precisely position the sheath before releasing the valve stent as in the prior art. It is only necessary to release the second stent into the ventricular region on the second side of the heart valve annulus. After the second stent is released, the position of the sheath is fine-tuned so that the second positioning portion of the second stent is positioned on the second side of the heart valve annulus. Then, the first stent can be released directly, ensuring that the first positioning portion and the second positioning portion are precisely positioned on the first side and the second side of the heart valve annulus, respectively. This reduces the positioning difficulty for releasing the prosthetic valve and improves the convenience of the release operation.

In some embodiments, the first stent is provided with a first connection hole, and the connection portion is provided with a second connection hole; and the prosthetic valve stent further comprises a connector configured to connect the first stent and the second stent, and the connector is passed through the first connection hole and the second connection hole.

In the above embodiments, by providing the first connection hole on the first stent and the second connection hole on the connection portion, and connecting by passing the connector through the first connection hole and the second connection hole, the first stent and the second stent can be processed separately during production, reducing the manufacturing difficulty of the prosthetic valve stent. During final assembly, the first stent and the second stent can be connected using the connector, which is convenient and quick to operate.

In some embodiments, an upstream direction and a downstream direction are defined according to a direction in which blood passes through the flow channel, and the first positioning portion is disposed at an upstream end of the first stent body.

In some embodiments, the first positioning portion comprises a plurality of first positioning units arranged along a circumference, and each of the first positioning units comprises two first positioning rods; for the two first positioning rods belonging to the same first positioning unit, ends of the two first positioning rods away from the flow channel are connected to form a first positioning point, and the first positioning points of the plurality of first positioning units are arranged at intervals along the circumference; and for the two first positioning rods belonging to two adjacent first positioning units, ends of the two first positioning rods close to the flow channel converge at a first node.

In the above embodiments, by arranging the plurality of first positioning units along the circumference, the plurality of first positioning units can cooperate with each other to achieve positioning on the circumference of the first side of the heart valve annulus. Each first positioning unit includes two first positioning rods. The two first positioning rods are connected at the ends away from the flow channel to form a first positioning point. Multi-point positioning of the first side of the heart valve annulus is achieved using the first positioning points in each first positioning unit. The structure is simple and the positioning stability is high.

In some embodiments, the first positioning portion further comprises a plurality of second positioning units, and one second positioning unit is disposed between two adjacent first positioning units; each of the second positioning units comprises two second positioning rods, ends of the two second positioning rods away from the flow channel are connected to form a second positioning point, the first positioning points and the second positioning points are alternately arranged along the circumference; and ends of the second positioning rods close to the flow channel are connected to the first positioning rods.

In the above embodiments, by disposing the second positioning unit between two adjacent first positioning units, connecting the ends of the two second positioning rods in the second positioning unit away from the flow channel to form the second positioning point, and arranging the first positioning points and the second positioning points alternately along the circumference, the number of positioning points of the first positioning portion on the circumference of the first side of the heart valve annulus is increased, which makes the positioning points between the first positioning portion and the first side of the heart valve annulus denser, reducing the force on each positioning point as a single point. Moreover, the second positioning unit relies on the first positioning unit, and the two are connected as an integral body, which has stronger integrity and improves the stability of the first positioning portion.

In some embodiments, the first positioning portion further comprises a plurality of third positioning units arranged along a circumference, and each of the third positioning units comprises two third positioning rods; for the two third positioning rods belonging to the same third positioning unit, ends of the two third positioning rods away from the flow channel are connected to form a third positioning point, the third positioning points of the plurality of third positioning units are arranged at intervals along the circumference, and the first positioning point protrudes in a direction away from the flow channel relative to the third positioning point; and for the two third positioning rods belonging to two adjacent third positioning units, ends of the two third positioning rods close to the flow channel converge at the first node.

In the above embodiments, by further including the plurality of third positioning units in the first positioning portion, including two third positioning rods in each third positioning unit, and connecting the two third positioning rods at the ends away from the flow channel to form the third positioning point, the number of positioning points between the first positioning portion and the first side of the heart valve annulus is increased. Furthermore, the first positioning point protrudes in the direction away from the flow channel relative to the third positioning point, that is, the third positioning point is located on an inner side of the first positioning point, so that the first positioning portion and the first side of the heart valve annulus achieve multi-point positioning in the radial direction, improving the positioning stability between the first positioning portion and the first side of the heart valve annulus. In addition, for the two third positioning rods belonging to two adjacent third positioning units, ends of the two third positioning rods close to the flow channel converge at the first node, so that the first positioning unit and the third positioning unit are connected as a whole, improving the integrity of the first positioning portion.

In some embodiments, the first stent body comprises a plurality of support units arranged along a circumference to enclose and form the flow channel; each of the support units comprises a first support rod and a second support rod; in a same support unit, an upstream end of the first support rod and an upstream end of the second support rod are respectively connected to two first nodes, and a downstream end of the first support rod and a downstream end of the second support rod are connected to form a second node.

In the above embodiments, in the plurality of support units, the upstream end of the first support rod and the upstream end of the second support rod are respectively connected to two first nodes, so that the plurality of support units and the first positioning portion are connected as a whole.

In some embodiments, the second node is provided with a first connection hole configured to connect the second stent.

In the above embodiments, since the second node is located at the downstream end of the first support rod and the downstream end of the second support rod, and the second node is provided with the first connection hole connecting the second stent, this is equivalent to the second stent being connected to the downstream end of the first stent. This is more conducive to the situation that after the second stent is released, the second stent only drives a downstream end portion of the first stent to expand, ensuring that after the second stent is fully released, the upstream end of the first stent remains located within the sheath.

In some embodiments, the number of the first positioning units is twice the number of the support units; in a same support unit, the upstream end of the first support rod and the upstream end of the second support rod are respectively connected to two first nodes that are spaced apart.

In some embodiments, each of the support units further comprises a third support rod and a fourth support rod; one end of the third support rod is connected to the first node located between the first support rod and the second support rod, and the other end is connected to a middle portion of the first support rod; and one end of the fourth support rod is connected to the first node located between the first support rod and the second support rod, and the other end is connected to a middle portion of the second support rod.

In the above embodiments, through the provision of the third support rod and the second support rod, one end of the third support rod is connected to the first node located between the first support rod and the second support rod, so that all the first nodes in the first positioning unit can correspondingly support and connect to the support unit, avoiding the phenomenon of a suspended first node and ensuring the overall stability of the first stent.

In some embodiments, the connection portion is located on a radial outer side of the first stent body, the second positioning portion extends radially outward from the connection portion and is located upstream of the connection portion, and the second positioning portion comprises a plurality of fourth positioning units arranged along a circumference.

In the above embodiments, the second positioning portion includes a plurality of fourth positioning units. By positioning with the second side of the heart valve annulus through the plurality of circumferentially distributed fourth positioning units, multi-point positioning is achieved, avoiding weak positioning points on the circumference of the second stent and ensuring the positioning stability of the second stent.

In some embodiments, each of the fourth positioning units comprises two fourth positioning rods; for the two fourth positioning rods belonging to a same fourth positioning unit, ends of the two fourth positioning rods away from the flow channel are connected to form a fourth positioning point, and the fourth positioning points of the plurality of fourth positioning units are arranged at intervals along the circumference; for the two fourth positioning rods belonging to two adjacent fourth positioning units, ends of the two fourth positioning rods close to the flow channel converge at a third node.

In the above embodiments, each fourth positioning unit includes two fourth positioning rods. The two fourth positioning rods are connected at the ends away from the flow channel to form a fourth positioning point. Multi-point positioning of the second side of the heart valve annulus is achieved using the fourth positioning points in each fourth positioning unit. The structure of the fourth positioning unit is simple and the positioning stability is high.

In some embodiments, the second positioning portion further comprises a plurality of fifth positioning units arranged along a circumference, the fifth positioning units are located downstream of the fourth positioning units, and each of the fifth positioning units comprises two fifth positioning rods; for the two fifth positioning rods belonging to a same fourth positioning unit, ends of the two fifth positioning rods away from the flow channel are connected to form a fifth positioning point, and the fifth positioning points of the plurality of fifth positioning units are arranged at intervals along the circumference; for the two fifth positioning rods belonging to two adjacent fifth positioning units, ends of the two fifth positioning rods close to the flow channel converge at a fourth node.

In the above embodiments, the second positioning portion further includes a plurality of circumferentially distributed fifth positioning units, and the fifth positioning units are located downstream of the fourth positioning units. The two fifth positioning rods in the fifth positioning unit are connected at the ends away from the flow channel to form the fifth positioning point, so that the second stent has multi-point positioning in the extension direction of the flow channel, improving the positioning effect and positioning stability of the second stent with the second side of the heart valve annulus.

In some embodiments, the connection portion comprises a plurality of fifth support rods arranged at intervals along a circumference, and one end of each of the fifth support rods is connected to the third node and the other end is connected to the fourth node.

In the above embodiments, the fourth positioning unit and the fifth positioning unit are connected as a whole through the fifth support rod, and the second positioning portion and the connection portion are connected as a whole, ensuring the integrity of the second stent.

In some embodiments, the fourth node is provided with a second connection hole configured to connect the first stent.

In the above embodiments, since the fourth node is located at the downstream end of the fifth support rod, and the fourth node is provided with the second connection hole for connecting the first stent, this is equivalent to the downstream end of the second stent being connected to the downstream end of the first stent. This is more conducive to the situation that after the second stent is released, the second stent can be fully released, and the release of the second stent only drives a downstream end portion of the first stent to expand, ensuring that after the second stent is fully released, the upstream end of the first stent remains located within the sheath.

In some embodiments, the number of the first positioning units is six, and the six first positioning units are distributed at equal intervals along the circumference.

Embodiments of the present invention further provide a prosthetic valve. The prosthetic valve includes a valve and the prosthetic valve stent. The valve is located in the flow channel of the first stent body.

In some embodiments, the prosthetic valve comprises a skirt, the skirt is disposed on an outer side of the second stent and/or the first stent, and an inner side of the skirt extends to the valve.

In the above embodiments, by disposing the skirt on the outer side of the second stent, and extending the inner side of the skirt to the valve, the skirt covers and shields the outer side of the second stent and/or the first stent, which can prevent blood on the second side of the heart valve annulus from flowing back to the first side of the heart valve annulus, ensuring that blood flows only from the first side of the heart valve annulus to the second side of the heart valve annulus.

Embodiments of the present invention further provide a method for releasing a prosthetic valve. The release method includes the following steps: releasing a second stent of the prosthetic valve to radially expand the second stent, and adjusting the second stent to position a second positioning portion at a second side of a target annular object; and releasing a first stent of the prosthetic valve to position a first positioning portion of the first stent at a first side of the target annular object.

The prosthetic valve stent allows the second stent to self-expand while the first stent is not fully released. The second stent may not completely fit the second side of the target annular object, and after the second stent is released and before the upstream end of the first stent is released, the position of the prosthetic valve stent can be adjusted. Since the position of the second stent can be fine-tuned after release, a certain deviation is allowed for the second stent to fit the second side of the target annular object, reducing the positioning difficulty for releasing the prosthetic valve and improving the convenience of the release operation. After adjusting the position of the sheath, with the aid of the second positioning portion of the already released second stent positioned on the second side of the heart valve annulus, the first stent can be released directly, ensuring that the first positioning portion and the second positioning portion are precisely positioned on the first side and the second side of the target annular object, respectively. This completes the precise release of the prosthetic valve stent, reducing the difficulty of the surgery and also reducing the risk of the surgery.

In some embodiments, adjusting the second stent refers to adjusting an angle of the second stent relative to the target annular object; and the step of positioning the second positioning portion on the second side of the target annular object refers to pulling the second stent to cause the second positioning portion to approach and fit closely against the target annular object. In this way, when adjusting the position of the second stent, both the angle of the second stent relative to the target annular object and the distance between the second stent and the target annular object can be adjusted, thereby achieving precise positioning of the second stent with the target annular object.

Other features and advantages of the present invention will be described in detail in the subsequent specific embodiment section.

### BRIEF DESCRIPTION OF THE DRAWINGS

In order to more clearly illustrate the technical solutions of the embodiments of the present invention, the drawings used in the embodiments will be briefly introduced below. It should be understood that the following drawings show only certain embodiments of the present invention and should therefore not be considered as limiting the scope. For those of ordinary skill in the art, other related drawings can be obtained based on these drawings without creative efforts.
FIG. 1 is a schematic structural view illustrating positioning of a prosthetic valve stent provided by some embodiments of the present invention at a heart valve annulus;
FIG. 2 is a schematic structural view of a prosthetic valve stent provided by some embodiments of the present invention;
FIG. 3 is a schematic structural view of the prosthetic valve stent provided by some embodiments of the present invention from another angle;
FIG. 4 is a top view of the prosthetic valve stent in FIG. 3;
FIG. 5 is a top view of a first stent provided by some embodiments of the present invention;
FIG. 6 is a front view of a first stent provided by some embodiments of the present invention;
FIG. 7 is a schematic structural view of a second stent provided by some embodiments of the present invention;
FIG. 8 is a front view of the second stent in FIG. 7;
FIG. 9 is a schematic view of a state before release of a prosthetic valve provided by some embodiments of the present invention;
FIG. 10 is a schematic structural view after release of a second stent in a prosthetic valve provided by some embodiments of the present invention;
FIG. 11 is a schematic view of a state where the second stent is positioned at a heart valve annulus after adjusting the position of a sheath in FIG. 10;
FIG. 12 is a schematic structural view after completion of release of a prosthetic valve provided by some embodiments of the present invention;
FIG. 13 is a schematic structural view of fine-tuning an angle before release of a prosthetic valve provided by some embodiments of the present invention; and
FIG. 14 is a schematic view of a state of fine-tuning after release of a second stent of the prosthetic valve provided by some embodiments of the present invention.

Reference Numerals: 10 - first stent; 11 - first stent body; 110 - support unit; 111 - first support rod; 112 - second support rod; 113 - second node; 1130 - first connection hole; 114 - third support rod; 115 - fourth support rod; 12 - first positioning portion; 121 - first positioning unit; 1210 - first positioning rod; 1211 - first positioning point; 1212 - first node; 122 - second positioning unit; 1220 - second positioning rod; 1221 - second positioning point; 123 - third positioning unit; 1230 - third positioning rod; 1231 - third positioning point; 20 - second stent; 21 - connection portion; 210 - fifth support rod; 22 - second positioning portion; 221 - fourth positioning unit; 2210 - fourth positioning rod; 2211 - fourth positioning point; 2212 - third node; 222 - fifth positioning unit; 2220 - fifth positioning rod; 2221 - fifth positioning point; 2222 - fourth node; 2223 - second connection hole; 30 - flow channel; 100 - prosthetic valve stent; 200 - heart valve annulus; 201 - first side of the heart valve annulus; 202 - second side of the heart valve annulus; 300 - sheath.

### DETAILED DESCRIPTION OF EMBODIMENTS

In order to make the objects, technical solutions, and advantages of the embodiments of the present invention be more clearly understood, the technical solutions in the embodiments of the present invention will be clearly and completely described below with reference to the drawings in the embodiments of the present invention. Obviously, the described embodiments are only a part of the embodiments of the present invention, rather than all of the embodiments. Generally, the components of the embodiments of the present invention described and illustrated in the drawings herein may be arranged and designed in various different configurations.

Therefore, the detailed description of the embodiments of the present invention provided in the drawings is not intended to limit the scope of the claimed application, but merely represents selected embodiments of the present invention. All other embodiments obtained by a person of ordinary skill in the art based on the embodiments in the present invention without creative efforts shall fall within the scope of protection of the present invention.

It should be noted that: like reference numerals and letters refer to like items in the following drawings, so once an item is defined in one drawing, it does not need to be further defined and explained in subsequent drawings.

In the description of the embodiments of the present invention, it should be noted that the indication of orientation or positional relationship is based on the orientation or positional relationship shown in the drawings, or the orientation or positional relationship in which the product of the application is usually placed when used, only for the convenience of describing the present invention and simplifying the description, rather than indicating or implying that the device or element must have a specific orientation, be constructed and operated in a specific orientation, and therefore cannot be understood as a limitation to the present invention. In addition, the terms "first," "second," "third," etc. are used only for distinguishing description and cannot be understood as indicating or implying relative importance.

In the description of the present invention, it should also be noted that, unless otherwise explicitly specified and limited, the terms "set" and "connection" should be understood in a broad sense. For example, "connection" may be a fixed connection, a detachable connection, or an integral connection; it may be a direct connection, or an indirect connection through an intermediate medium; and it may be the internal communication of two elements. For those of ordinary skill in the art, the specific meanings of the above terms in the present invention can be understood according to specific situations.

### Embodiments

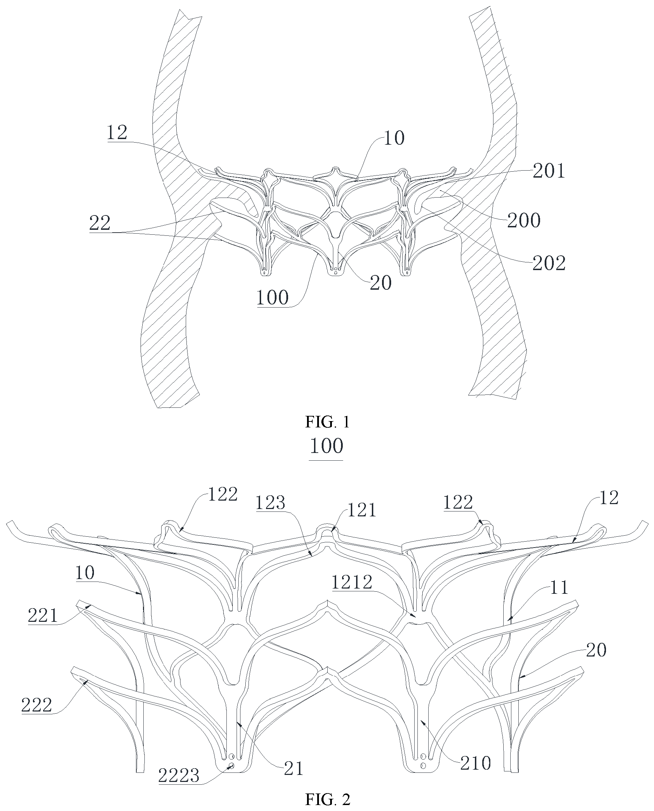

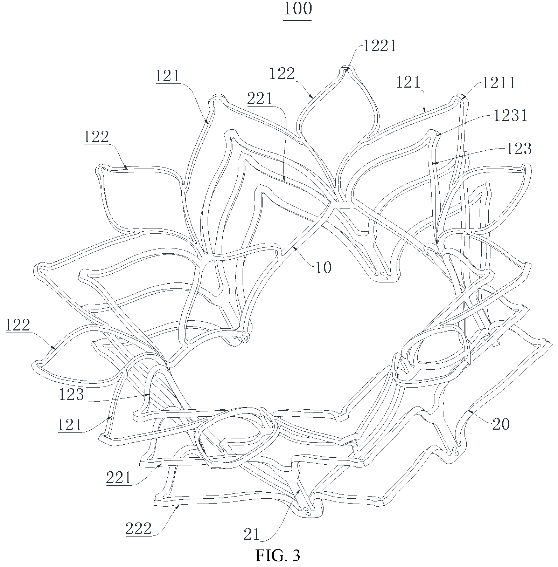

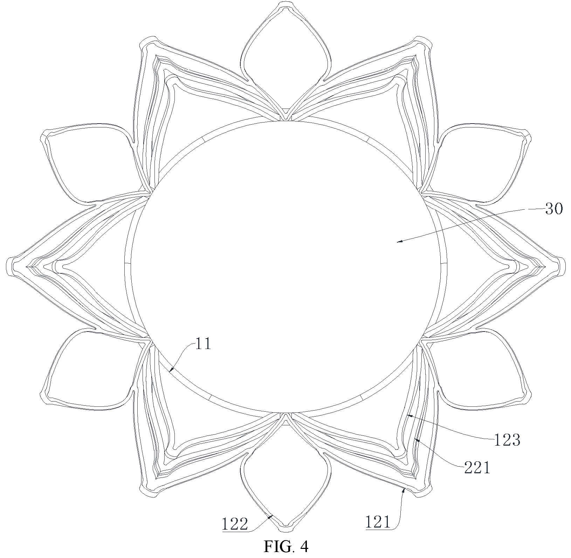

Embodiments of the present invention provide a prosthetic valve stent. Referring to FIG. 1 to FIG. 14, the prosthetic valve stent 100 includes a first stent 10 and a second stent 20. The first stent 10 includes a first stent body 11 and a first positioning portion 12 configured to be positioned with a first side 201 of a heart valve annulus. The first stent body 11 defines a flow channel 30 for blood flow. The first positioning portion 12 extends radially outward from the first stent body 11. The second stent 20 includes a connection portion 21 located on a radial outer side of the first stent body 11 and connected to the first stent body 11, and a second positioning portion 22 configured to be positioned with a second side 202 of the heart valve annulus.

In the present embodiment, by adopting a double-layer stent structure for the prosthetic valve stent 100, designing the positioning structure of the prosthetic valve stent 100 as a split-type structure, and providing a first positioning portion 12 and a second positioning portion 22 respectively on the first stent 10 and the second stent 20, during the release of the prosthetic valve, the second stent 20 can be released first. At this time, the first stent 10 has not yet been fully released, and the position of the second stent 20 can be adjusted according to the actual situation. After the second positioning portion 22 of the second stent 20 is positioned on the second side 202 of the heart valve annulus, the first stent 10 is then released to complete the release of the prosthetic valve stent 100. In this manner, before releasing the second stent 20, after the sheath 300 enters the target position, it is not necessary to precisely position the sheath 300 before releasing the prosthetic valve stent as in the prior art. It is only necessary to release the second stent 20 into the ventricular region on the second side 202 of the heart valve annulus. After the second stent 20 is released, the position of the sheath 300 is fine-tuned so that the second positioning portion 22 of the second stent 20 is positioned on the second side 202 of the heart valve annulus. Then, the first stent 10 can be released directly, ensuring that the first positioning portion 12 and the second positioning portion 22 are precisely positioned on the first side 201 and the second side 202 of the heart valve annulus, respectively. This reduces the positioning difficulty for releasing the prosthetic valve and improves the convenience of the release operation.

The heart valve annulus 200 includes a first side 201 of the heart valve annulus and a second side 202 of the heart valve annulus. It can be understood that the first side 201 of the heart valve annulus is the atrial side of the heart valve annulus 200, and the second side 202 of the heart valve annulus is the ventricular side of the heart valve annulus 200. The blood flow direction is from the first side 201 of the heart valve annulus to the second side 202 of the heart valve annulus, that is, from the atrial side of the heart valve annulus 200 to the ventricular side of the heart valve annulus 200.

In some embodiments, the first stent 10 is provided with a first connection hole 1130, and the connection portion 21 is provided with a second connection hole 2223. The prosthetic valve stent 100 further includes a connector configured to connect the first stent 10 and the second stent 20. The connector is passed through the first connection hole 1130 and the second connection hole 2223. By providing the first connection hole 1130 on the first stent 10 and the second connection hole 2223 on the connection portion 21, and connecting the first stent 10 and the second stent 20 by passing the connector through the first connection hole 1130 and the second connection hole 2223, the first stent 10 and the second stent 20 can be processed separately during production, reducing the manufacturing difficulty of the prosthetic valve stent 100. During final assembly, the first stent 10 and the second stent 20 can be connected using the connector, which is convenient and quick to operate.

The connection between the first stent 10 and the second stent 20 may be a rigid connection or a flexible connection. For example, the connector may be a rivet, a screw, etc. The connector passes through the first connection hole 1130 and the second connection hole 2223 to rigidly connect the first stent 10 and the second stent 20. Alternatively, the connector is a drawstring or a soft rope, etc., and the first stent 10 and the second stent 20 are flexibly connected by passing the drawstring or the soft rope through the first connection hole 1130 and the second connection hole 2223 and tying a knot. Certainly, the specific connector can be determined according to the actual situation.

In addition, the first positioning portion 12 on the first stent body 11 can be located at any position such as an upstream end or a middle portion of the first stent body 11.

Exemplarily, an upstream direction and a downstream direction are defined according to the direction in which blood passes through the flow channel 30. The first positioning portion 12 is disposed at the upstream end of the first stent body 11.

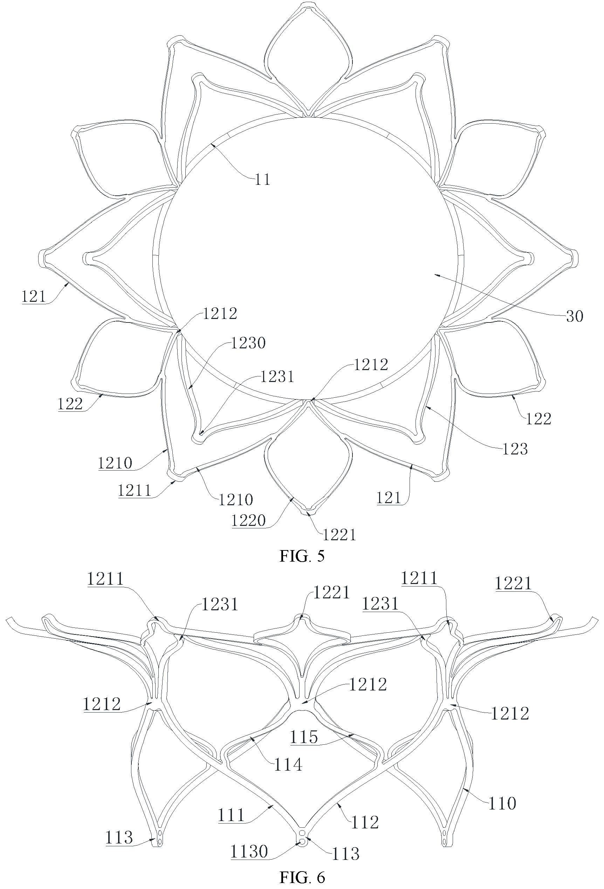

In some embodiments, referring to FIG. 5 and FIG. 6, the first positioning portion 12 includes a plurality of first positioning units 121. The plurality of first positioning units 121 is arranged circumferentially. Each first positioning unit 121 includes two first positioning rods 1210. For the two first positioning rods 1210 belonging to the same first positioning unit 121, ends of the two first positioning rods 1210 away from the flow channel 30 are connected to form a first positioning point 1211. The first positioning points 1211 of the plurality of first positioning units 121 are arranged at intervals along the circumference. For the two first positioning rods 1210 belonging to two adjacent first positioning units 121, ends of the two first positioning rods 1210 close to the flow channel 30 converge at a first node 1212. By arranging the plurality of first positioning units 121 along the circumference, the plurality of first positioning units 121 can cooperate with each other to achieve positioning on the circumference of the first side 201 of the heart valve annulus. Each first positioning unit 121 includes two first positioning rods 1210. The two first positioning rods 1210 are connected at the ends away from the flow channel 30 to form a first positioning point 1211. The multi-point positioning of the first side 201 of the heart valve annulus is achieved using the first positioning points 1211 in each first positioning unit 121. The structure is simple and the positioning stability is high.

The number of the plurality of first positioning units 121 may be four, five, or six, etc. In the present embodiment, the number of the first positioning units 121 is six, and the six first positioning units 121 are distributed at equal intervals along the circumference. In addition, the first positioning rod 1210 may be a straight rod or a curved rod. In the present embodiment, the first positioning rod 1210 is a curved rod.

In some embodiments, continuing to refer to FIG. 5 and FIG. 6, the first positioning portion 12 further includes a plurality of second positioning units 122. One second positioning unit 122 is disposed between two adjacent first positioning units 121. Each second positioning unit 122 includes two second positioning rods 1220. Ends of the two second positioning rods 1220 away from the flow channel 30 are connected to form a second positioning point 1221. The first positioning points 1211 and the second positioning points 1221 are arranged alternately along the circumference. Ends of the second positioning rods 1220 close to the flow channel 30 are connected to the first positioning rods 1210. By disposing the second positioning unit 122 between two adjacent first positioning units 121, connecting the ends of the two second positioning rods 1220 away from the flow channel 30 in the second positioning unit 122 to form the second positioning point 1221, and arranging the first positioning points 1211 and the second positioning points 1221 alternately along the circumference, the number of positioning points of the first positioning portion 12 on the circumference of the first side 201 of the heart valve annulus is increased. This makes the positioning points of the first positioning portion 12 and the first side 201 of the heart valve annulus denser, reducing the force on each positioning point as a single point. Moreover, the second positioning unit 122 relies on the first positioning unit 121, and the two are connected as an integral body, which has stronger integrity and improves the stability of the first positioning portion 12.

In the case where the number of the first positioning units 121 is six, the number of the second positioning units 122 is also six. In addition, the second positioning rod 1220 may be a straight rod or a curved rod. In the present embodiment, the second positioning rod 1220 is a curved rod.

In some embodiments, continuing to refer to FIG. 5 and FIG. 6, the first positioning portion 12 further includes a plurality of third positioning units 123. The plurality of third positioning units 123 are arranged circumferentially. Each third positioning unit 123 includes two third positioning rods 1230. For the two third positioning rods 1230 belonging to the same third positioning unit 123, ends of the two third positioning rods 1230 away from the flow channel 30 are connected to form a third positioning point 1231. The third positioning points 1231 of the plurality of third positioning units 123 are arranged at intervals along the circumference. The first positioning point 1211 protrudes in a direction away from the flow channel 30 relative to the third positioning point 1231. For the two third positioning rods 1230 belonging to two adjacent third positioning units 123, ends of the two third positioning rods 1230 close to the flow channel 30 converge at a first node 1212.

By further including the plurality of third positioning units 123 in the first positioning portion 12, including two third positioning rods 1230 in each third positioning unit 123, and connecting the two third positioning rods 1230 at the ends away from the flow channel 30 to form the third positioning point 1231, the number of positioning points between the first positioning portion 12 and the first side 201 of the heart valve annulus is increased. Furthermore, the first positioning point 1211 protrudes in the direction away from the flow channel 30 relative to the third positioning point 1231, that is, the third positioning point 1231 is located on an inner side of the first positioning point 1211, so that the first positioning portion 12 and the first side 201 of the heart valve annulus achieve multi-point positioning in the radial direction, improving the positioning stability between the first positioning portion 12 and the first side 201 of the heart valve annulus. In addition, for the two third positioning rods 1230 belonging to two adjacent third positioning units 123, ends of the two third positioning rods 1230 close to the flow channel 30 converge at the first node 1212, so that the first positioning unit 121 and the third positioning unit 123 are connected as a whole, improving the integrity of the first positioning portion 12.

In the case where the number of the first positioning units 121 is six, the number of the third positioning units 123 is also six. Moreover, the third positioning rod 1230 may be a straight rod or a curved rod. In the present embodiment, the third positioning rod 1230 is a curved rod.

In some embodiments, referring to FIG. 6, the first stent body 11 includes a plurality of support units 110. The plurality of support units 110 are arranged circumferentially to enclose and form the flow channel 30. Each support unit 110 includes a first support rod 111 and a second support rod 112. In the same support unit 110, an upstream end of the first support rod 111 and an upstream end of the second support rod 112 are respectively connected to two first nodes 1212, and a downstream end of the first support rod 111 and a downstream end of the second support rod 112 are connected to form a second node 113. In the plurality of support units 110, the upstream end of the first support rod 111 and the upstream end of the second support rod 112 are respectively connected to two first nodes 1212, so that the plurality of support units 110 and the first positioning portion 12 are connected as a whole.

The first support rod 111 and the second support rod 112 may be straight rods or curved rods. In the present embodiment, the first support rod 111 and the second support rod 112 are curved rods.

In some embodiments, continuing to refer to FIG. 6, the second node 113 is provided with the first connection hole 1130 for connecting the second stent 20. Since the second node 113 is located at the downstream end of the first support rod 111 and the downstream end of the second support rod 112, and the second node 113 is provided with the first connection hole 1130 connecting the second stent 20, this is equivalent to the second stent 20 being connected to the downstream end of the first stent 10. This is more conducive to the situation that after the second stent 20 is released, the second stent 20 only drives a downstream end portion of the first stent 10 to expand, ensuring that after the second stent 20 is fully released, the upstream end of the first stent 10 remains located within the sheath 300.

The number of the first connection holes 1130 may be one or more. In the present embodiment, the number of the first connection holes 1130 is two.

In some embodiments, the number of the first positioning units 121 is twice the number of the support units 110. In the same support unit 110, the upstream end of the first support rod 111 and the upstream end of the second support rod 112 are respectively connected to two first nodes 1212 that are spaced apart.

In the case where the number of the first positioning units 121 is six, the number of the support units 110 is three.

In addition, since the upstream end of the first support rod 111 and the upstream end of the second support rod 112 are respectively connected to two spaced apart first nodes 1212, there will be a suspended first node 1212 among the plurality of first nodes 1212.

In some embodiments, each support unit 110 further includes a third support rod 114 and a fourth support rod 115. One end of the third support rod 114 is connected to the first node 1212 located between the first support rod 111 and the second support rod 112, and the other end is connected to a middle portion of the first support rod 111. One end of the fourth support rod 115 is connected to the first node 1212 located between the first support rod 111 and the second support rod 112, and the other end is connected to a middle portion of the second support rod 112. Through the provision of the third support rod 114 and the second support rod 112, one end of the third support rod 114 is connected to the first node 1212 located between the first support rod 111 and the second support rod 112, so that all the first nodes 1212 in the first positioning unit 121 can correspondingly support and connect to the support unit 110, avoiding the phenomenon of a suspended first node 1212 and ensuring the overall stability of the first stent 10.

The third support rod 114 and the fourth support rod 115 may be straight rods or curved rods. In the present embodiment, the third support rod 114 and the fourth support rod 115 are curved rods.

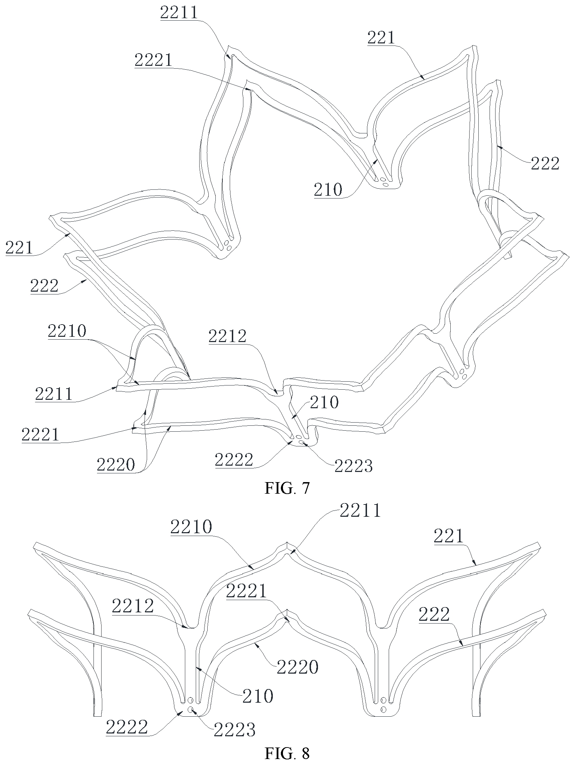

In some embodiments, referring to FIG. 7 and FIG. 8, the connection portion 21 is located on the radial outer side of the first stent body 11. The second positioning portion 22 extends radially outward from the connection portion 21 and is located upstream of the connection portion 21. The second positioning portion 22 includes a plurality of fourth positioning units 221. The plurality of fourth positioning units 221 are arranged circumferentially. The second positioning portion 22 includes a plurality of fourth positioning units 221, and by positioning with the second side 202 of the heart valve annulus via the plurality of circumferentially distributed fourth positioning units 221, multi-point positioning is achieved, avoiding weak positioning points on the circumference of the second stent 20 and ensuring the positioning stability of the second stent 20.

In some embodiments, referring to FIG. 7 and FIG. 8, each fourth positioning unit 221 includes two fourth positioning rods 2210. For the two fourth positioning rods 2210 belonging to the same fourth positioning unit 221, ends of the two fourth positioning rods 2210 away from the flow channel 30 are connected to form a fourth positioning point 2211. The fourth positioning points 2211 of the plurality of fourth positioning units 221 are arranged at intervals along the circumference. For the two fourth positioning rods 2210 belonging to two adjacent fourth positioning units 221, ends of the two fourth positioning rods 2210 close to the flow channel 30 converge at a third node 2212. Each fourth positioning unit 221 includes two fourth positioning rods 2210. The two fourth positioning rods 2210 are connected at the ends away from the flow channel 30 to form a fourth positioning point 2211. Multi-point positioning of the second side 202 of the heart valve annulus is achieved using the fourth positioning points 2211 in each fourth positioning unit 221. The structure of the fourth positioning unit 221 is simple and the positioning stability is high.

The fourth positioning rod 2210 may be a straight rod or a curved rod. In the present embodiment, the fourth positioning rod 2210 is a curved rod.

In some embodiments, continuing to refer to FIG. 7 and FIG. 8, the second positioning portion 22 further includes a plurality of fifth positioning units 222. The plurality of fifth positioning units 222 are arranged circumferentially. The fifth positioning units 222 are located downstream of the fourth positioning units 221. Each fifth positioning unit 222 includes two fifth positioning rods 2220. For the two fifth positioning rods 2220 belonging to the same fourth positioning unit 221, ends of the two fifth positioning rods 2220 away from the flow channel 30 are connected to form a fifth positioning point 2221. The fifth positioning points 2221 of the plurality of fifth positioning units 222 are arranged at intervals along the circumference. For the two fifth positioning rods 2220 belonging to two adjacent fifth positioning units 222, ends of the two fifth positioning rods 2220 close to the flow channel 30 converge at a fourth node 2222.

The second positioning portion 22 further includes a plurality of circumferentially distributed fifth positioning units 222, the fifth positioning units 222 are located downstream of the fourth positioning units 221, and the two fifth positioning rods 2220 in the fifth positioning unit 222 are connected at the ends away from the flow channel 30 to form the fifth positioning point 2221, so that the second stent 20 has multi-point positioning in the extension direction of the flow channel 30, improving the positioning effect and positioning stability of the second stent 20 with the second side 202 of the heart valve annulus.

The fifth positioning rod 2220 may be a straight rod or a curved rod. In the present embodiment, the fifth positioning rod 2220 is a curved rod.

In some embodiments, referring to FIG. 8, the connection portion 21 includes a plurality of fifth support rods 210. The plurality of fifth support rods 210 are arranged at intervals along the circumference. One end of each fifth support rod 210 is connected to the third node 2212, and the other end is connected to the fourth node 2222. The fourth positioning unit 221 and the fifth positioning unit 222 are connected as a whole through the fifth support rod 210, and the second positioning portion 22 and the connection portion 21 are connected as a whole, ensuring the integrity of the second stent 20.

In some embodiments, the fourth node 2222 is provided with the second connection hole 2223 for connecting the first stent 10. Since the fourth node 2222 is located at the downstream end of the fifth support rod 210, and the fourth node 2222 is provided with the second connection hole 2223 for connecting the first stent 10, this is equivalent to the downstream end of the second stent 20 being connected to the downstream end of the first stent 10. This is more conducive to the situation that after the second stent 20 is released, the second stent 20 can be fully released, and the release of the second stent 20 only drives a downstream end portion of the first stent 10 to expand, ensuring that after the second stent 20 is fully released, the upstream end of the first stent 10 remains located within the sheath 300.

In the case where the number of the first connection holes 1130 on the second node 113 is set to two, the number of the second connection holes 2223 on the fourth node 2222 corresponds thereto, and the number is also two.

Embodiments of the present invention provide a prosthetic valve. The prosthetic valve includes a valve (not shown in the drawings) and the prosthetic valve stent 100. The valve is located in the flow channel 30 of the first stent body 11.

In some embodiments, the prosthetic valve includes a skirt (not shown in the drawings). The skirt is disposed on an outer side of the second stent 20 and/or the first stent 10. An inner side of the skirt extends to the valve. By disposing the skirt on the outer side of the second stent 20 with the inner side of the skirt extending to the valve, the skirt covers and shields the outer side of the second stent 20 and/or the first stent 10, which can prevent blood on the second side 202 of the heart valve annulus from flowing back to the first side 201 of the heart valve annulus, ensuring that blood flows only from the first side 201 of the heart valve annulus to the second side 202 of the heart valve annulus.

In the present embodiment, the skirt is disposed on the outer side of the second stent 20, and the inner side of the skirt extends to a lower edge of the valve.

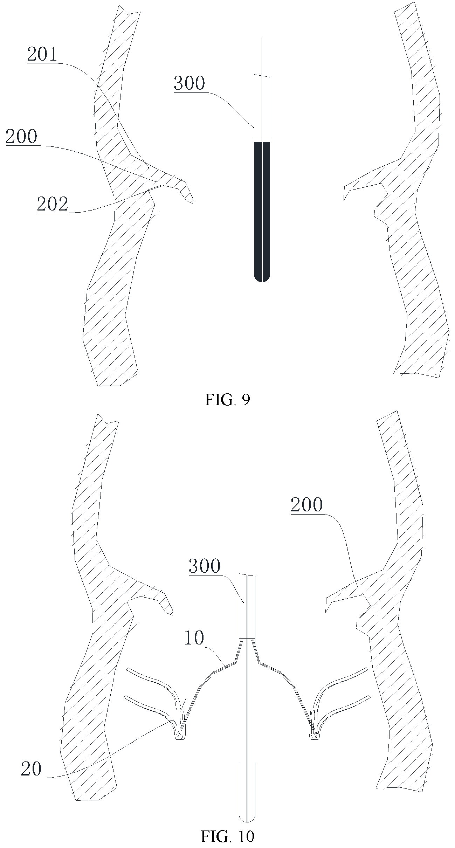

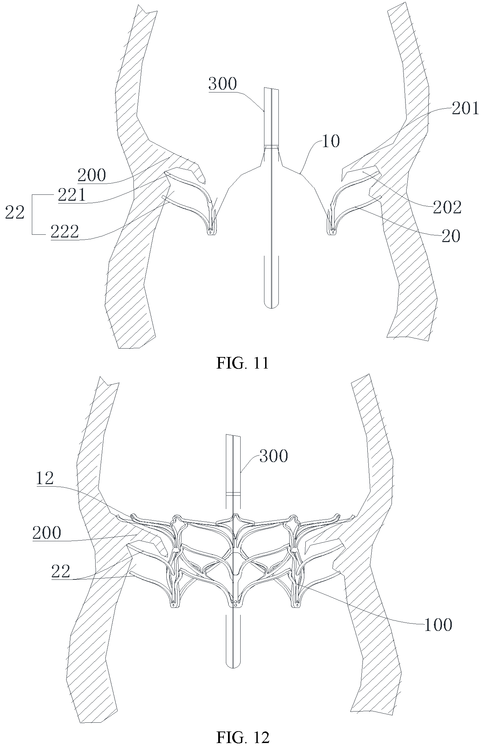

Embodiments of the present invention provide a method for releasing a prosthetic valve. The release method includes the following steps: referring to FIG. 9 and FIG. 10, releasing the second stent 20 of the prosthetic valve to radially expand the second stent 20; referring to FIG. 11, adjusting the second stent 20 so that the second positioning portion 22 is positioned on the second side of a target annular object; referring to FIG. 12, releasing the first stent 10 of the prosthetic valve so that the first positioning portion 12 of the first stent 10 is positioned on the first side of the target annular object.

It should be noted that the release method can be used on animal cadavers, animals, or human models to test the release status, release force, etc. of the prosthetic valve. The above-mentioned target annular object may be a heart valve annulus in an animal cadaver or a human model, such as a mitral valve annulus or a tricuspid valve annulus, etc. In the present embodiment, the target annular object is a mitral valve annulus, which is referred to hereinafter as the heart valve annulus. It can be understood that the first side of the target annular object, i.e., the first side 201 of the heart valve annulus, is also the side of the heart valve annulus 200 close to the atrium; the second side of the target annular object, i.e., the second side 202 of the heart valve annulus, is also the side of the heart valve annulus 200 close to the ventricle.

The prosthetic valve stent 100 allows the second stent 20 to self-expand while the first stent 10 is not fully released. The second stent 20 may not completely fit the second side 202 of the heart valve annulus. After the second stent 20 is released and before the upstream end of the first stent 10 is released, the position of the prosthetic valve stent 100 can be adjusted. Since the second stent 20 can be fine-tuned after release, a certain deviation is allowed for the second stent 20 to fit the second side 202 of the heart valve annulus, reducing the positioning difficulty for releasing the prosthetic valve and improving the convenience of the release operation. After adjusting the position of the sheath 300, with the aid of the second positioning portion 22 of the already released second stent 20 positioned on the second side 202 of the heart valve annulus, the first stent 10 can be released directly, and this ensures that the first positioning portion 12 and the second positioning portion 22 are precisely positioned on the first side 201 and the second side 202 of the heart valve annulus, respectively. This completes the precise release of the prosthetic valve stent 100, reducing the difficulty of the surgery and also reducing the risk of the surgery.

In some embodiments, adjusting the second stent 20 refers to adjusting an angle of the second stent 20 relative to the heart valve annulus 200. The step of causing the second positioning portion 22 to be positioned on the second side 202 of the heart valve annulus refers to pulling the second stent 20 to make the second positioning portion 22 approach and fit closely against the heart valve annulus 200. In this way, when adjusting the position of the second stent 20, both the angle of the second stent 20 relative to the heart valve annulus 200 and the distance between the second stent 20 and the heart valve annulus 200 can be adjusted, thereby achieving precise positioning of the second stent 20 with the second side 202 of the heart valve annulus.

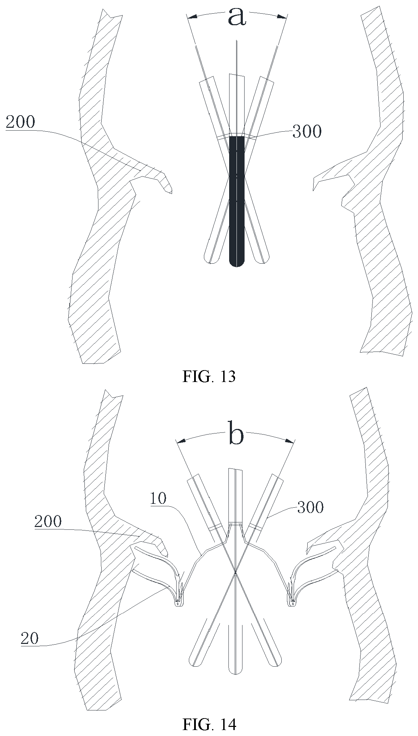

Specifically, as shown in FIG. 13, since the second stent 20 can be fine-tuned after release, a certain deviation is allowed for the second stent 20 to perfectly fit the second side 202 of the heart valve annulus. Therefore, during the release process, the sheath 300 relative to the plane of the heart valve annulus 200 may have a certain angle. As shown in FIG. 7, the allowable angle is symmetrically centered on the plane perpendicular to the heart valve annulus, and its maximum allowable amount a is 50°. Therefore, the requirement for positioning accuracy at this time is reduced, meaning the operation is simpler and improving the popularity of the structure of this prosthetic valve stent 100. At the same time, the time used for positioning before the release of the second stent 20 can be shortened, because adjusting the position after releasing the second stent 20 before it completely fits the second side 202 of the heart valve annulus is simpler and takes less time than the positioning operation before release.

After the second stent 20 is released and the adjustment of the second stent 20 is completed, the second stent 20 fits the second side 202 of the heart valve annulus, which has a positioning effect for the smooth fitting of the first stent 10 to the first side 201 of the heart valve annulus after release. After the second stent 20 is released first and fits the second side 202 of the heart valve annulus, the self-expanding process of the first stent 10 after the first stent 10 releases can better fit the first side 201 of the heart valve annulus under the pulling action of the second stent 20. As shown in FIG. 14, at this time, it can still be released accurately if there is a certain angular deviation between the sheath 300 and the plane where the heart valve annulus is located. Similarly, at this time, the allowable offset angle is centered on a direction perpendicular to the plane of the heart valve annulus, with an offset range of b, and this angle is greater than the above-mentioned angle a. Therefore, after the first step of completing the release of the second stent 20 and fitting the valve annulus plane, there is no need to adjust the angle of the sheath 300 relative to the plane where the heart valve annulus is located at this time, and the first stent 10 can be released directly, thereby completing the release and positioning of the prosthetic valve stent 100.

It should be noted that, in the case of no conflict, the features in the embodiments of the present invention can be combined with each other.

The above descriptions are only preferred embodiments of the present invention and are not intended to limit the present invention. For those skilled in the art, the present invention may have various modifications and variations. Any modifications, equivalent substitutions, improvements, etc. made within the spirit and principles of the present invention shall fall within the scope of protection of the present invention.

### INDUSTRIAL APPLICABILITY

The present invention provides a prosthetic valve stent, a prosthetic valve, and a release method, which reduce the release difficulty of the prosthetic valve and improve the convenience of the release operation.

In addition, it can be understood that the prosthetic valve stent, the prosthetic valve, and the release method of the present invention are reproducible and can be widely applied in the field of medical technology.

## Claims

1. A prosthetic valve stent, **characterized by** comprising:
a first stent, comprising a first stent body, and a first positioning portion configured to be positioned with a first side of a heart valve annulus, wherein the first stent body defines a flow channel for blood flow, and the first positioning portion extends radially outward from the first stent body; and
a second stent, comprising a connection portion located on a radial outer side of the first stent body and connected to the first stent body, and a second positioning portion configured to be positioned with a second side of the heart valve annulus.

2. The prosthetic valve stent according to claim 1, wherein the first stent is provided with a first connection hole, and the connection portion is provided with a second connection hole; and
the prosthetic valve stent further comprises a connector configured to connect the first stent and the second stent, and the connector is passed through the first connection hole and the second connection hole.

3. The prosthetic valve stent according to claim 1, wherein an upstream direction and a downstream direction are defined according to a direction in which blood passes through the flow channel, and the first positioning portion is disposed at an upstream end of the first stent body.

4. The prosthetic valve stent according to claim 3, wherein the first positioning portion comprises a plurality of first positioning units arranged along a circumference, and each of the first positioning units comprises two first positioning rods;
for the two first positioning rods belonging to a same first positioning unit, ends of the two first positioning rods away from the flow channel are connected to form a first positioning point, and the first positioning points of the plurality of first positioning units are arranged at intervals along the circumference; and
for the two first positioning rods belonging to two adjacent first positioning units, ends of the two first positioning rods close to the flow channel converge at a first node.

5. The prosthetic valve stent according to claim 4, wherein the first positioning portion further comprises a plurality of second positioning units, and one second positioning unit is disposed between two adjacent first positioning units; and
each of the second positioning units comprises two second positioning rods, ends of the two second positioning rods away from the flow channel are connected to form a second positioning point, the first positioning points and the second positioning points are alternately arranged along the circumference; and ends of the second positioning rods close to the flow channel are connected to the first positioning rods.

6. The prosthetic valve stent according to claim 5, wherein the first positioning portion further comprises a plurality of third positioning units arranged along the circumference, and each of the third positioning units comprises two third positioning rods;
for the two third positioning rods belonging to a same third positioning unit, ends of the two third positioning rods away from the flow channel are connected to form a third positioning point, the third positioning points of the plurality of third positioning units are arranged at intervals along the circumference, and the first positioning point protrudes in a direction away from the flow channel relative to the third positioning point; and
for the two third positioning rods belonging to two adjacent third positioning units, ends of the two third positioning rods close to the flow channel converge at the first node.

7. The prosthetic valve stent according to claim 4, wherein the first stent body comprises a plurality of support units arranged along the circumference to enclose and form the flow channel; and
each of the support units comprises a first support rod and a second support rod; in a same support unit, an upstream end of the first support rod and an upstream end of the second support rod are respectively connected to two first nodes, and a downstream end of the first support rod and a downstream end of the second support rod are connected to form a second node.

8. The prosthetic valve stent according to claim 7, wherein the second node is provided with a first connection hole configured to connect the second stent.

9. The prosthetic valve stent according to claim 7, wherein a number of the first positioning units is twice a number of the support units; and
in a same support unit, the upstream end of the first support rod and the upstream end of the second support rod are respectively connected to two first nodes that are spaced apart.

10. The prosthetic valve stent according to claim 9, wherein each of the support units further comprises a third support rod and a fourth support rod;
one end of the third support rod is connected to the first node located between the first support rod and the second support rod, and the other end is connected to a middle portion of the first support rod; and
one end of the fourth support rod is connected to the first node located between the first support rod and the second support rod, and the other end is connected to a middle portion of the second support rod.

11. The prosthetic valve stent according to claim 1, wherein the connection portion is located on a radial outer side of the first stent body, the second positioning portion extends radially outward from the connection portion and is located upstream of the connection portion, and the second positioning portion comprises a plurality of fourth positioning units arranged along a circumference.

12. The prosthetic valve stent according to claim 11, wherein each of the fourth positioning units comprises two fourth positioning rods;
for the two fourth positioning rods belonging to a same fourth positioning unit, ends of the two fourth positioning rods away from the flow channel are connected to form a fourth positioning point, and the fourth positioning points of the plurality of fourth positioning units are arranged at intervals along the circumference; and
for the two fourth positioning rods belonging to two adjacent fourth positioning units, ends of the two fourth positioning rods close to the flow channel converge at a third node.

13. The prosthetic valve stent according to claim 12, wherein the second positioning portion further comprises a plurality of fifth positioning units arranged along the circumference, the fifth positioning units are located downstream of the fourth positioning units, and each of the fifth positioning units comprises two fifth positioning rods;
for the two fifth positioning rods belonging to a same fourth positioning unit, ends of the two fifth positioning rods away from the flow channel are connected to form a fifth positioning point, and the fifth positioning points of the plurality of fifth positioning units are arranged at intervals along the circumference; and
for the two fifth positioning rods belonging to two adjacent fifth positioning units, ends of the two fifth positioning rods close to the flow channel converge at a fourth node.

14. The prosthetic valve stent according to claim 13, wherein the connection portion comprises a plurality of fifth support rods arranged at intervals along the circumference, and one end of each of the fifth support rods is connected to the third node and the other end is connected to the fourth node.

15. The prosthetic valve stent according to claim 13, wherein the fourth node is provided with a second connection hole configured to connect the first stent.

16. The prosthetic valve stent according to any one of claims 4-10, wherein a number of the first positioning units is six, and the six first positioning units are distributed at equal intervals along the circumference.

17. A prosthetic valve, **characterized by** comprising a valve and the prosthetic valve stent according to any one of claims 1-16, wherein the valve is located in the flow channel of the first stent body.

18. The prosthetic valve according to claim 17, wherein the prosthetic valve comprises a skirt, the skirt is disposed on an outer side of the second stent and/or the first stent, and an inner side of the skirt extends to the valve.

19. A method for releasing the prosthetic valve according to claim 16 or 17, **characterized by** comprising following steps:
(a) releasing a second stent of the prosthetic valve to radially expand the second stent, and adjusting the second stent to position a second positioning portion at a second side of a target annular object; and
(b) releasing a first stent of the prosthetic valve to position a first positioning portion of the first stent at a first side of the target annular object.

20. The method for releasing the prosthetic valve according to claim 19, wherein adjusting the second stent in step (a) refers to adjusting an angle of the second stent relative to the target annular object; and the step of positioning the second positioning portion on the second side of the target annular object refers to pulling the second stent to cause the second positioning portion to approach and fit closely against the target annular object.
